# EUROPÄISCHE PATENTSCHRIFT

(11) **EP 2 397 070 B1**
(45) Veröffentlichungstag und Bekanntmachung des Hinweises auf die Patenterteilung: **13.10.2021**
(21) Anmeldenummer: 11168235.7
(22) Anmeldetag: 31.05.2011
(51) Int. Cl.: A61B 3/16, A61B 3/107

(54) **Analyse des intraokularen Drucks**
Analysis of intraocular pressure
Analyse de la pression intra-oculaire

(30) Priorität: 28.10.2010 DE 102010049634; 28.10.2010 DE 102010049633; 21.06.2010 EP 10166681
(43) Veröffentlichungstag der Anmeldung: 21.12.2011
(73) Patentinhaber: Oculus Optikgeräte GmbH, 35582 Wetzlar-Dutenhofen (DE)
(72) Erfinder: Steinmüller, Andreas, 35435 Wettenberg (DE)
(74) Vertreter: advotec.

(56) Entgegenhaltungen:
- EP-A1- 1 692 998
- DE-A1-102006 039 893
- US-A- 6 120 444
- US-A1- 2005 030 473
- US-A1- 2007 121 120
- KANEKO M ET AL: "Dynamic Sensing of Human Eye", ROBOTICS AND AUTOMATION, 2005. PROCEEDINGS OF THE 2005 IEEE INTERNATIONAL CONFERENCE ON BARCELONA, SPAIN 18-22 APRIL 2005, PISCATAWAY, NJ, USA,IEEE, 18. April 2005 (2005-04-18), Seiten 2871-2876, XP010871696, DOI: 10.1109/ROBOT.2005.1570549 ISBN: 978-0-7803-8914-4
- KEMPF R ET AL: "Understanding eye deformation in non-contact tonometry", CONFERENCE PROCEEDINGS. ANNUAL INTERNATIONAL CONFERENCE OF THE IEEE ENGINEERING IN MEDICINE AND BIOLOGY SOCIETY (IEEE CAT. NO. 06CH37748); 30 AUG.-3 SEPT. 2006; NEW YORK, NY, USA, IEEE, PISCATAWAY, NJ, USA, 30. August 2006 (2006-08-30), Seiten 5428-5431, XP031390671, ISBN: 978-1-4244-0032-4
- None

## Beschreibung

Die Erfindung betrifft ein ophthalmologisches Analyseverfahren zur Messung eines intraokularen Drucks in einem Auge mit einem Analysesystem, sowie ein derartiges Analysesystem mit den Merkmalen der Ansprüche 1 und 16.

Analyseverfahren und -systeme sind hinreichend bekannt und dienen primär einer möglichst genauen, berührungslosen Messung eines intraokularen Drucks in einem Auge. Dazu wird beispielsweise ein Non-Kontakt-Tonometer verwandt, mit Hilfe dessen ein Luftstoß auf das zu untersuchende Auge appliziert wird, wobei eine Stärke des Luftstoßes so gewählt wird, dass die Hornhaut des Auges unter Ausbildung einer konkaven Oberflächenform eingedrückt wird. Vor Erreichen eines Maximums einer Verformung der Hornhaut bzw. vor einem Einklappen der Hornhaut in Richtung der Augenlinse, bildet die Hornhaut kurzzeitig eine plane Fläche aus, die als sogenannter erster Applanationspunkt bezeichnet wird. Nach der maximalen Auslenkung der Hornhaut und einem Zurückklappen derselben in den ursprünglichen Zustand, wird ein ebensolcher zweiter Applanationspunkt von der Hornhaut durchlaufen. Durch eine Inbezugsetzung eines Drucks des Luftstoßes mit einem zeitlichen Verlauf der Applanation der Hornhaut ist es nun möglich, einen intraokularen Druck zu ermitteln. Die mit dem Non-Kontakt-Tonometer ermittelten Messwerte werden in Relation zu Vergleichsmesswerten gesetzt, welche mit einem relativ genauer messenden Applanationstonometer bzw. Kontakt-Tonometer ermittelt wurden, so dass als ein Ergebnis ein dem tatsächlichen intraokularen Druck angenäherter Augeninnendruck abgeleitet werden kann.

Jedoch ist ein mit einem Non-Kontakt-Tonometer gemessener intraokularer Druck gegenüber einer Druckmessung mit einem Applanationstonometer noch nicht hinreichend genau, da eine Messung unter anderem von der Hornhaut verfälscht wird. Um eine Messgenauigkeit zu verbessern, wurde daher versucht, den Einfluss der Hornhaut auf die Messung zu berücksichtigen, beispielsweise durch eine Dickenmessung oder eine Messung von Hornhautradien vor Beginn der Non-Kontakt-Tonometermessung. Auch ist es bekannt, einen Elastizitätsmodul bzw. Youngschen-Modul als eine biomechanische Materialeigenschaft der Hornhaut zu berücksichtigen und die betreffende Messung mit einem entsprechenden Berechnungsfaktor zu korrigieren. Dabei wird bei allen Messungen, auch bei unterschiedlichen Augen, davon ausgegangen, dass der Elastizitätsmodul immer gleich groß und somit konstant ist. Weiter wird davon ausgegangen, dass eine Hornhaut einen in allen Bereichen der Hornhaut gleich großen Elastizitätsmodul aufweist. Eine derartige Einbeziehung eines Elastizitätsmoduls in einer Non-Kontakt-Tonometermessung hat den Nachteil, dass diese Materialeigenschaft bzw. dieser Materialkennwert zur Charakterisierung einer Zugbelastung dient, wie Sie bei einer Non-Kontakt-Tonometermessung nicht auftritt. Weiter variiert ein Elastizitätsmodul von Auge zu Auge individuell und auch, in Abhängigkeit von den jeweiligen Hornhautbereichen, innerhalb der Hornhaut selbst. Eine Einbeziehung derartiger Materialkennwerte und eine Berechnung des Messergebnisses können daher noch nicht zu befriedigend genauen Messergebnissen führen. Weiter ist es bekannt, die biomechanischen Eigenschaften einer Hornhaut während einer Non-Kontakt-Tonometermessung in diese mit einzubeziehen bzw. diese so bereits während der Messung zu ermitteln. Dazu wird ein Luftstoß auf eine Hornhaut aufgebracht, wobei ein Pumpendruck während des Verlaufs der Messung mittels eines Druckaufnehmers kontinuierlich erfasst wird. Weiter wird ein zeitlicher Verlauf der Messung erfasst sowie ein erster und ein zweiter Applanationspunkt der Hornhaut optisch detektiert. Ein intraokularer Druck kann nun beispielsweise durch eine Bestimmung der zum Zeitpunkt der ersten und zweiten Applanation herrschenden Drücke abgeleitet werden, insbesondere da die zur Biegung der Hornhaut notwendigen Kräfte beim Ein- bzw. Ausklappen der Hornhaut als gleich groß angenommen werden und sich somit gegenseitig aufheben. Folglich resultiert ein intraokularer Druck aus einem Mittelwert der zum Einklappen und Ausklappen der Hornhaut aufgewendeten Kraft, aufgebracht durch den Luftstoß.

Alternativ ist es bekannt, eine Hysterese zwischen dem erstem und dem zweitem Applanationspunkt zu bestimmen und auf Basis der Hysteresemessung den intraokularen Druck abzuleiten bzw. zu korrigieren. Bei der Hysteresemessung wird der erste und zweite Applanationspunkt optisch detektiert und zum Zeitverlauf einer Druckkurve einer Pumpe in Relation gesetzt, das heißt für jeden Applanationspunkt wird ein zugehöriger Zeitwert sowie ein Druckwert ermittelt. Da ein Einklappen der Hornhaut bzw. ein Erreichen des ersten Applanationspunktes bei einem höheren Druckwert erfolgt, als ein Ausklappen bzw. ein Erreichen des zweiten Applanationspunktes, kann dieser Druckunterschied zur Bestimmung der Hysterese als eine Materialeigenschaft der Hornhaut herangezogen werden.

Nachteilig bei diesen Messverfahren ist, dass eine durch einen Luftstoß bewirkte Bewegung der Hornhaut dynamischen Effekten unterliegt, welche derartige Zeit-/Druckmessungen verfälschen können, insbesondere da die dynamischen Effekte bei den beschriebenen Non-Kontakt-Tonometermessungen nicht berücksichtigt werden können. Um derartige, unerwünschte Schwingungen der Hornhaut zu vermeiden, wird eine Geschwindigkeit des Luftstoßes soweit als Möglich minimiert, um ein Messergebnis nicht durch eine unerwünschte Hornhautbewegung zu verfälschen. Weiter ist es notwendig eine Synchronität zwischen Beginn des Luftstoßes und der erforderlichen Zeitmessung herzustellen. Durch eine mechanische, zur Ausbildung eines Luftstoßes verwendeten Pumpe, wie beispielsweise eine Kolbenpumpe, kann diese Zeitsynchronität jedoch, beispielsweise infolge von Massenträgheit oder ―reibung, nicht genau erreicht und somit ein Messergebnis verfälscht werden. Auch wird, wie zuvor bereits erwähnt, der Luftstoß drucküberwacht, das heißt nach Bedarf im Verlauf der Messung beeinflusst. So wird nach einem Überschreiten des ersten Applanationspunktes der Luftstoß vermindert bzw. abgeschaltet, um ein übermäßiges Eindrücken der Hornhaut zu verhindern. Dazu ist jedoch ebenfalls eine kontinuierliche Drucküberwachung eines Pumpendrucks sowie eine Überwachung eines zeitlichen Verlaufs desselben relativ zu den Zeitpunkten der ersten und zweiten Applanation notwendig, was eine Einbeziehung einer Reihe von möglichen, einer Messung verfälschenden Fehlerquellen, zur Folge hat. Insgesamt sind daher die aus dem Stand der Technik bekannten Analyseverfahren und ―systeme mit voneinander abhängiger, paralleler Druck- und Zeitmessung bei gleichzeitiger Detektion der Applanationspunkte gegenüber einer Messung mit einem Kontakt-Tonometer noch vergleichsweise ungenau.

Die EP 1 692 998 A1 beschreibt ein Non-Kontakt-Tonometer und eine Verwendung eines Scheimpflugsystems zur Gewinnung von Schnittbildern der Hornhaut sowie eine Analyseeinrichtung mittels der aus Bildinformationen bzw. den Schnittbildern der der Hornhaut der intraokulare Druck eines Auges abgeleitet werden kann.

Der Artikel Dynamic Sensing of Human Eye, veröffentlicht im Rahmen der International Conference on Robotics and Automation, beschreibt eine Berücksichtigung einer Steifigkeit und einer Dämpfung einer Hornhaut in Verbindung mit einer Messung eines Augeninnendrucks. Die Augeninnendruckmessung erfolgt dabei mit einem Non-Kontakt-Tonometer, wobei eine Verformung der Hornhaut mit einer Hochgeschwindigkeitskamera aufgenommen wird. Die Hochgeschwindigkeitskamera ist offensichtlich orthogonal zu einer Sehachse positioniert.

Die US 6,120,444 betrifft ein ophthalmologisches Analysegerät, welches aus einem Non-Kontakt-Tonometer und einem Placidosystem gebildet ist. Insbesondere kann auch ein Scheimpflugsystem vorgesehen sein. Aus jeweils gewonnenen Bildinformationen wird eine intraokulare Spannung abgeleitet, wobei die Bildinformationen mit in einer Datenbank gespeicherten Bildinformationen verglichen werden und auf Basis des Vergleichs die Spannung berechnet wird.

Aus der DE 10 2006 039 893 A1 ist ein Non-Kontakt-Tonometer bzw. ein Verfahren zum Messen eines intraokularen Drucks bekannt, bei dem ein Druckverlauf eines Tonometers in Relation zu Applanationspunkten des Auges gestellt wird. Aus einer Lage der Applanationspunkte auf einer Zeitachse relativ zum Druckverlauf, wird ein Einfluss der Hornhaut auf einen Augeninnendruck berechnet und dieser damit korrigiert.

Die US 2007/0121120 A1 offenbart ein ophthalmologisches Analysegerät, welches unter anderem einen Laser umfasst. Mittels des Gerätes wird eine Hornhaut in Schwingung versetzt, wobei aus einer Geschwindigkeit einer Oberfläche des Auges ein intraokularer Druck abgeleitet wird.

Die US 2005/0030473 A1 beschreibt ein Verfahren zur Messung eines intraokularen Drucks eines Auges mittels eines ophthalmologischen Analysegerätes. Bei der Berechnung eines intraokularen Drucks wird insbesondere ein Korrekturfaktor herangezogen, der einen Widerstand der Hornhaut gegen eine Auslenkung beschreiben soll und der in Abhängigkeit einer Dicke und von viskoelastischen Eigenschaften der Hornhaut variiert. Weiter kann eine diesbezügliche Steifigkeit der Hornhaut aus einer mit einem Non-Kontakt-Tonometer auf die Hornhaut aufgebrachten Kraft bzw. einem Luftstoß und einer dadurch erzeugten Applanationsfläche abgeleitet werden. Eine Verformung des Auges wird mit einer Hochgeschwindigkeitskamera aufgenommen, die seitlich des Auges angeordnet ist.

Der Artikel Understanding eye deformation in non-contact tonometry, veröffentlicht im Rahmen der EMBS Annual International Conference, beschreibt seitliche Videoaufnahme einer mit einem Non-Kontakt-Tonometer verformten Hornhaut eines Auges. Insbesondere wird ein Einfluss einer Steifigkeit der Hornhaut auf die Messergebnisse erwähnt.

Der vorliegenden Erfindung liegt daher die Aufgabe zugrunde, ein ophthalmologisches Analyseverfahren zur Messung eines intraokularen Drucks in einem Auge sowie ein derartiges Analysesystem vorzuschlagen, mit dem eine vergleichsweise verbesserte Messgenauigkeit erzielbar ist.

Diese Aufgabe wird durch ein ophthalmologisches Analyseverfahren mit den Merkmalen des Anspruchs 1 und ein Analysesystem mit den Merkmalen des Anspruchs 16 gelöst.

Bei dem erfindungsgemäßen, ophthalmologischen Analyseverfahren zur Messung eines objektiven intraokularen Drucks in einem Auge mit einem Analysesystem, umfasst das Analysensystem eine Betätigungseinrichtung, mit der eine Hornhaut des Auges berührungsfrei verformt wird, wobei mit der Betätigungseinrichtung ein Luftstoß zur Verformung der Hornhaut auf das Auge aufgebracht wird, ein Beobachtungssystem, mit dem die Verformung der Hornhaut beobachtet und aufgenommen wird, wobei das Beobachtungssystem eine Kamera und eine Beleuchtungseinrichtung in einer Scheimpfluganordnung umfasst, wobei mit der Kamera Schnittbilder der unverformten und verformten Hornhaut aufgenommen werden, und eine Analyseeinrichtung, mit der aus den Schnittbildern der Hornhaut der objektive intraokulare Druck abgeleitet wird, wobei in der Analyseeinrichtung aus den Schnittbildern der Hornhaut eine Materialeigenschaft der Hornhaut abgeleitet wird, wobei als eine Materialeigenschaft eine Steifigkeit der Hornhaut abgeleitet wird, wobei eine maximale Amplitude eines geometrischen Verformungsverlaufs der Hornhaut in Richtung einer Sehachse des Auges oder einer Geräteachse des Analysesystems aus den Schnittbildern der Hornhaut abgeleitet wird, wobei eine Funktion der Steifigkeit für einen gemessenen subjektiven intraokularen Druck und die gemessene maximale Amplitude einer Datenbank des ophthalmologischen Analysesystems entnommen wird, und wobei der objektive intraokulare Druck unter Berücksichtigung der Steifigkeit der Hornhaut abgeleitet wird.

Der Begriff der Steifigkeit ist hier explizit nicht als ein Elastizitätsmodul bzw. Youngscher-Modul zu verstehen, sondern als eine Materialeigenschaft, die durch eine auf das Auge wirkenden Druckbelastung charakterisiert ist bzw. dieser entgegensteht, also den bei einer Tonometermessung tatsächlich auftretenden Lastfall betrifft. Die Steifigkeit ist somit eine richtungsabhängige Kenngröße des Materials der Hornhaut. Weiter ist die Steifigkeit durch das Material der Hornhaut selbst und nicht durch sonstige, äußere Einflüsse bestimmt. Auch wirken im Material der Hornhaut intrinsische Spannungen die die Steifigkeit der Hornhaut beeinflussen.

Gemäß der Erfindung sind der intraokulare Druck und die Steifigkeit der Hornhaut als eine die Hornhaut beschreibende Materialeigenschaft getrennt voneinander bestimmbar. So wird während einer einzigen Messung durch Aufbringen eines Luftstoßes nach einem herkömmlichen, tonometrischen Verfahren ein erster intraokularer Druck bestimmt. Parallel dazu wird aus der Verformung der Hornhaut, die während der Verformung durch das Beobachtungssystem aufgenommen wird, die Steifigkeit der Hornhaut abgeleitet. Da die Steifigkeit der Hornhaut einen wesentlichen Einfluss auf ein Verformungsverhalten der Hornhaut bzw. die Messung des ersten intraokularen Drucks des Auges hat, kann der Einfluss der Hornhaut auf die Messung des ersten intraokularen Drucks berücksichtigt werden. So wird der zuvor gemessene, erste intraokulare Druck um den Einfluss der Hornhaut auf die Messung korrigiert, so dass ein objektiver intraokularer Druck als ein Ergebnis der Messung abgeleitet wird. Die Steifigkeit der Hornhaut ist dabei im Wesentlichen näherungsweise eine lineare Funktion von dem ersten, gemessenen subjektiven Intraokularen Druck des Auges sowie einer gemessenen maximalen Amplitude der Verformung der Hornhaut. Bei einem Graph der Funktion der Steifigkeit ist der subjektive intraokulare Druck beispielsweise auf einer Ordinatenachse und die maximale Amplitude der Verformung auf einer Abzissenachse abgebildet, wobei die Steifigkeit dann im Wesentlichen eine Form einer Geraden mit negativer Steigung aufweist. Je nach Messwert für die Abzissenachse und Ordinatenachse ergibt sich bei sich verändernden Messwerten im Wesentlichen eine Parallelverschiebung der Geraden, woraus jeweils unterschiedliche Steifigkeiten resultieren. Der objektive intraokulare Druck wird von der ermittelten Steifigkeit abgeleitet bzw. kann von der Geraden der Steifigkeit aus einem Schnittpunkt des Wertes für den subjektiven intraokularen Drucks bzw. des Wertes für die maximale Amplitude mit der Geraden für die Steifigkeit abgetragen werden. Bei der Messung wird immer die Steifigkeit der Hornhaut als eine Materialeigenschaft für jede Messung neu ermittelt, dass heißt, es wird nicht, wie aus dem Stand der Technik bekannt, von einer für jedes beliebige Auge konstanten Materialeigenschaft ausgegangen. Weiter ist es besonders vorteilhaft, wenn während der Messung bzw. des Verformungsvorgangs der Hornhaut eine Serie bzw. Vielzahl von Schnittbildern der Hornhaut aufgenommen wird. So wird es möglich, eine Verformung der Hornhaut im Detail zu verfolgen und mittels einer Bildverarbeitung der Schnittbilder aus dem Verformungsablauf die entsprechende Materialeigenschaft bzw. einen objektiven intraokularen Druck abzuleiten.

Weiter ist zu beachten, dass bei dem erfindungsgemäßen Verfahren eine Druckmessung eines Pumpendrucks nicht notwendig ist. So wird jede beliebige Messung eines intraokularen Drucks immer mit dem gleichen, konstanten Pumpendruck durchgeführt. Da hier keine Variation einer Höhe des Pumpendrucks bzw. eine Zeitsynchronisation des Pumpendrucks erfolgen muss, können eine Reihe von möglichen Fehlerquellen ausgeschlossen und eine besonders genaue Messung durchgeführt werden. Im Übrigen kann die so bestimmte Materialeigenschaft der Hornhaut auch im Rahmen der refraktiven Augenchirurgie genutzt werden, um beispielsweise bei einem LASIK-Eingriff auf die jeweilige Hornhauteigenschaften abzustimmen.

Erfindungsgemäß umfasst das Beobachtungssystem eine Kamera und eine Beleuchtungseinrichtung in einer Scheimpfluganordnung, wobei mittels der Kamera die Schnittbilder aufgenommen werden. Das heißt die Kamera ist relativ zu einer optischen Achse einer Spaltbeleuchtungseinrichtung zur Beleuchtung des Auges in einer Scheimpfluganordnung angeordnet sein, so dass ein beleuchtetes Querschnittsbild des Auges mit der Kamera aufgenommen werden kann. Eine Kamera kann beispielsweise auch als eine Hochgeschwindigkeitskamera verwendet werden, die mindestens 4000 Bilder pro Sekunde aufnehmen kann. Auch fällt die optische Achse der Spaltbeleuchtungseinrichtung in eine Sehachse des Auges bzw. stimmt mit dieser überein. Vorzugsweise kann dann auch eine Wirkrichtung des Luftstoßes koaxial zur optischen Achse der Spaltbeleuchtungseinrichtung verlaufen.

Erfindungsgemäß wird zur Ableitung der Materialeigenschaft der Hornhaut eine Amplitude der Verformung der Hornhaut aus den Schnittbildern der Hornhaut abgeleitet. So kann der genaue geometrische Verlauf der Verformung leicht nachvollzogen werden. Das heißt, es kann zu jedem Zeitpunkt der Verformung die zu diesem Zeitpunkt existente, geometrische Gestalt der Verformung aufgenommen werden, so dass der geometrische Verlauf der Verformung in Art eines Films der Verformung erfasst werden kann. Beispielsweise ist auch ein Nachschwingen der Hornhaut nach einem Ausklappen bzw. dem zweiten Applanationspunkt so gut erfassbar.

Erfindungsgemäß wird die Steifigkeit als eine vom intraokularen Druck unabhängige Materialeigenschaft der Hornhaut abgeleitet. So können dann der intraokulare Druck und die Steifigkeit der Hornhaut als eine unabhängige, die Hornhaut beschreibende Materialeigenschaft besonders genau getrennt voneinander bestimmt werden.

Weiter ist es vorteilhaft, wenn ein Pumpendruck zur Erzeugung des Luftstoßes relativ zu einer Zeitdauer desselben in Form einer Glockenkurve verläuft. So kann der Pumpendruck für jede individuelle Messung identisch und vollkommen unbeeinflusst in Form des Luftstoßes auf die Hornhaut einwirken. Die Glockenkurve kann dabei unter anderem eine symmetrische Gestalt aufweisen.

Auch kann ein maximaler Pumpendruck zur Erzeugung des Luftstoßes bei vorangehenden und nachfolgenden Messungen gleich sein. So kann eine besonders gute Vergleichbarkeit von verschiedenen Messungen ermöglicht werden. Der maximale Pumpendruck kann beispielsweise 70 mm Hg betragen.

Um einen Pumpendruck dennoch gegebenenfalls korrigieren zu können bzw. um einen gewünschten Druckverlauf zu überprüfen, kann ein Pumpendruck zur Erzeugung des Luftstoßes bei Erreichen eines Applanationspunktes der Hornhaut gemessen werden. Beispielsweise kann eine Pumpe über einen Drucksensor verfügen, der die Überwachung des Pumpendrucks während der gesamten Messung ermöglicht. Dadurch können mögliche Fehler hinsichtlich des Pumpendrucks bei der Messung ausgeschlossen und eine Kontinuität aufeinanderfolgender Messungen sichergestellt werden.

Zur Ableitung der Materialeigenschaft kann auch ein Zeitabschnitt zwischen Beginn und Ende der Verformung der Hornhaut gemessen werden. So können insbesondere alle aufgenommenen Schnittbilder jeweils einem bestimmten Zeitpunkt der Messung zugeordnet werden, wodurch ein zeitlicher Ablauf der Verformung nachvollziehbar wird. Insbesondere kann ein Zeitpunkt der ersten und der zweiten Applanation der Hornhaut und somit ein zeitlicher Abstand genau bestimmt werden. So kann auch die Ermittlung dieses Zeitabschnittes zur Bestimmung der betreffenden Materialeigenschaft ausreichend sein. Weiter kann zur Ableitung der Materialeigenschaft ein Zeitabschnitt der gesamten Verformung der Hornhaut herangezogen werden.

Zur Ableitung der Materialeigenschaft kann eine Geschwindigkeit der bewegten Hornhaut gemessen werden. Wenn insbesondere der Zeitverlauf einer Verformung der Hornhaut bekannt ist, kann so auch eine Dynamik der Verformung untersucht werden, um besondere dynamische Effekte bei der Verformung in Hinblick auf die betreffende Materialeigenschaft zu bewerten. Beispielsweise kann sich ein Nachschwingen der Hornhaut bei einem Luftstoß somit nicht mehr verfälschend auf ein Messergebnis auswirken, wenn das Nachschwingen bei der Messung berücksichtigt wird. Auch wird so eine Geschwindigkeit eines Luftstoßes in Bezug auf sonst unerwünschte dynamische Effekte für eine Messung beliebig wählbar. Auch ist es möglich von der gemessenen Geschwindigkeit auf eine Eindrücktiefe bzw. maximale Amplitude zu schließen, da zwischen diesen Größen ein funktionaler Zusammenhang besteht.

Um die Materialeigenschaft noch genauer zu bestimmen, kann zur Ableitung der Materialeigenschaft eine maximale Verformung der Hornhaut aus den Schnittbildern der Hornhaut abgeleitet werden. Folglich kann eine maximale Eindrücktiefe der Hornhaut aus den Schnittbildern der Hornhaut ermittelt werden, wobei ergänzend auch ein Zeitpunkt der maximalen Verformung der Hornhaut zumindest relativ zu einem der Applanationspunkte festgestellt werden kann.

Zur noch genaueren Ableitung der Materialeigenschaft kann eine Krümmung der unverformten und/oder verformten Hornhaut aus den Schnittbildern der Hornhaut abgeleitet werden. Da die Schnittbilder der Hornhaut auch eine Geometrie derselben beschreiben, insbesondere vor Aufbringen des Luftstoßes, kann die Geometrie der Hornhaut in Verbindung mit der betreffenden Materialeigenschaft der Hornhaut in die Berechnung des objektiven, intraokularen Drucks mit einbezogen werden. Das heißt die Krümmungsradien bzw. eine Kurvatur der Hornhaut kann auf einer äußeren und/oder inneren Hornhautoberfläche aus den Schnittbildern mittels Bildverarbeitung abgeleitet werden. Dabei können die Krümmungsradien bei der unverformten Hornhaut als ein Korrekturfaktor und beispielsweise die Dicke der Hornhaut bei der verformten Hornhaut in die Messung einfließen und als ein Indikator für die Materialeigenschaft herangezogen werden.

Optional kann auch zur Ableitung einer weiteren Materialeigenschaft bei Erreichen eines Applanationspunktes der Hornhaut eine Größe einer ebenen Applanationsfläche gemessen werden. Beispielsweise kann eine Größe der Applanationsfläche bzw. deren Durchmesser und/oder deren Form als ein Indikator für eine Steifigkeit der Hornhaut berücksichtigt werden. Bei der Verformung der Hornhaut mittels des Luftstoßes kann dann es zu einer vollständigen Applanation der Hornhaut kommen, wobei eine erste Applanationsfläche mit dem Durchmesser d₁ ausgebildet wird. Die Applanationsfläche ist dann im Wesentlichen eben und liegt im Bereich einer Applanationsebene orthogonal zu einer Sehachse des Auges bzw. einer Geräteachse eines Analysesystems. Während der Verformung der Hornhaut wird in der Hornhaut eine konkave Vertiefung ausgebildet, die sich von der ersten Applanationsfläche wesentlich unterscheidet. Ein Vergleich der von der ersten Applanationsfläche abweichenden Verformungsfläche der Vertiefung mit der ersten Applanationsfläche ermöglicht eine Definition der weiteren Materialeigenschaft der Hornhaut, da die Verformungsfläche auch in Abhängigkeit der weiteren Materialeigenschaft ausgebildet wird. Ein Referenzmaßstab für die Abweichung kann in diesem Fall die erste Applanationsfläche bzw. der Durchmesser d₁ der ersten Applanationsfläche sein. Wenn der Vergleich mit dem Durchmesser dₙ der Verformungsfläche der Hornhaut erfolgt, kann der Vergleich besonders einfach ausgeführt werden. Der Durchmesser dₙ kann insbesondere bei einer Verformungsbewegung der Hornhaut nach Passieren der ersten Applanationsfläche bzw. eines ersten Applanationspunktes besonders einfach bestimmt werden, da dann die Verformungsfläche eine konkave Gestalt annimmt. Weiter kann die Verformungsfläche bzw. der Durchmesser dₙ in einem bestimmten Zeitabschnitt der Verformung relativ zur ersten Applanationsfläche oder auch ein anderer, messbarer Punkt oder eine Position der Hornhaut während der Verformung zur Definition der abweichenden Verformungsfläche der Hornhaut herangezogen werden. Auch können die ermittelte Abweichung und die Relativwerte der betreffenden Durchmesser in einer Datenbank gespeichert sein und verglichen werden. So kann für die in der Datenbank gespeicherten Werte ein objektiver Augeninnendruck oder auch ein entsprechender Korrekturwert bekannt sein, so dass der objektive intraokulare Druck des gemessenen Auges unter Berücksichtigung der weiteren Materialeigenschaft der Hornhaut abgeleitet werden kann.

Zur Ableitung der weiteren Materialeigenschaft kann ein Durchmesser d₂ einer Verformungsfläche der Hornhaut bei einer maximalen Verformung der Hornhaut in Richtung einer Sehachse oder Geräteachse bestimmt werden. Die maximale Verformung der Hornhaut kann aus einer Serie von Schnittbildern der verformten Hornhaut bestimmt werden. So wird es möglich für jede Messung einen Zeitpunkt der Verformung bzw. eine Geometrie der Hornhaut zu definieren, der bzw. die als ein Vergleichsmaßstab zur ersten Applanationsfläche der Hornhaut dienen kann. Auch kann der Durchmesser d₂ dann einfach dadurch bestimmt werden, dass dieser als ein Abstand von zwei gegenüberliegenden Punkten in einer Längsschnittebene der Hornhaut im Zustand der maximalen Verformung definiert wird, wobei die Punkte jeweils die dem Analysesystem am nächsten zugewandten Punkte repräsentieren. Diese Punkte können einem Schnittbild entnommen werden und repräsentieren folglich den Durchmesser d₂ der maximalen Verformung bzw. Deformation der Hornhaut. Zur Ableitung der weiteren Materialeigenschaft kann ein Verhältnis zwischen dem Durchmesser d₁ der ersten Applanationsfläche der Hornhaut und einem Durchmesser d₃ einer zweiten Applanationsfläche der Hornhaut bestimmt werden. Während einer Verformung der Hornhaut mittels des Luftstoßes erfolgt ein Einklappen der Hornhaut unter Ausbildung der ersten Applanationsfläche bis hin zu einer maximalen Verformung der Hornhaut mit einer konkaven Vertiefung sowie nachfolgend ein Ausklappen der Hornhaut unter Ausbildung der zweiten, weitestgehend ebenen Applanationsfläche bis zu einem Erreichen der ursprünglichen Gestalt der Hornhaut. Die zweite Applanationsfläche stellt damit einen in den Schnittbildern gut erkennbaren, geometrischen Referenzpunkt dar, der zur Definition der weiteren Materialeigenschaft durch einen Vergleich mit der ersten Applanationsfläche herangezogen werden kann. Insbesondere durch eine mögliche Abweichung der Durchmesser der Applanationsflächen kann eine weitere Materialeigenschaft der Hornhaut definiert bzw. bestimmt werden. Auch können die an die jeweilige Applanationsfläche anschließenden Radien der Hornhaut als ein weiterer Indikator verwendet werden.

Die weitere Materialeigenschaft der Hornhaut kann noch weiter differenziert werden, wenn die Verformung der Hornhaut durch eine freie Schwingung der Hornhaut fortgesetzt wird, und wenn als eine weitere Materialeigenschaft dann die freie Schwingung der Hornhaut bestimmt wird. Ein Ausschwingen der Hornhaut nach Aufbringen des Luftstoßes und Erreichen der ursprünglichen Gestalt der Hornhaut unterscheidet sich regelmäßig bei verschiedenen Augen. Somit kann das Ausschwingen der Hornhaut als eine weitere Materialeigenschaft der Hornhaut definiert werden, anhand der ein intraokularer Druck korrigiert werden kann. Folglich kann vorgesehen sein, mittels des Beobachtungssystems Schnittbilder der Hornhaut über die eigentliche Verformung der Hornhaut hinaus aufzunehmen, um das Ausschwingen bzw. die freie Schwingung der Hornhaut zu ermitteln. Eine freie Schwingung der Hornhaut kann leicht dadurch bestimmt werden, dass eine Frequenz und/oder Amplitude der freien Schwingung gemessen wird. So wird es möglich die Frequenz und/oder eine Amplitudengröße und Abnahme während eines Ausschwingens zur Definition der weiteren Materialeigenschaft heranzuziehen.

Als eine Materialeigenschaft kann ein Schubmodul (G) der Hornhaut abgeleitet werden. Ein Schubmodul kann als eine lineare Materialkenngröße als eine besonders vereinfachte Angabe der Steifigkeit der Hornhaut verwendet werden, insbesondere da ein derartig lineares Materialverhalten unaufwendig im Rahmen der Analyseeinrichtung interpretiert werden kann.

Im Gegensatz dazu kann als eine Materialeigenschaft auch eine nichtlineare Steifigkeit der Hornhaut abgeleitet werden. Eine Berücksichtigung der nichtlinearen Steifigkeit kann zu wesentlich genaueren Messergebnissen führen, da hier alle auf die Hornhaut während der Verformung wirkenden Belastungsgrößen bzw. -werte berücksichtigt werden können. Eine Funktion der Steifigkeit selbst kann für jede einzelne Messung des intraokularen Drucks aus dem Verhältnis des gemessenen, subjektiven intraokularen Drucks und der maximalen Amplitude der Verformung jeweils errechnet werden. Alternativ ist es möglich, auf eine Funktion der Steifigkeit zurückzugreifen, die für den jeweiligen gemessenen subjektiven intraokularen Druck und die jeweilige, gemessene maximale Amplitude der Verformung in einer Datenbank verfügbar ist. Die in der Datenbank enthaltenen Funktionen der Steifigkeit können dabei aus einer Vielzahl von Versuchsreihen mit unterschiedlichen Druckmessungen verschiedener Augen ermittelt worden sein.

Als eine weitere Materialeigenschaft kann eine Spannung der Hornhaut abgeleitet werden, wobei Spannungen im Material der Hornhaut visualisiert werden, wobei der intraokulare Druck unter Berücksichtigung von weiteren Struktur und/oder Materialeigenschaften der Hornhaut abgeleitet wird. Eine weitere Materialeigenschaft ist im vorliegenden Zusammenhang als eine Eigenschaft definiert, die dem Material unabhängig von äußeren Einflüssen innewohnt. Eine Struktureigenschaft ist eine Eigenschaft, die durch äußere Einflüsse im Material oder auch durch eine Gestalt des Materials beeinflusst ist. So kann es vorgesehen sein, Spannungen der Hornhaut durch die Aufnahme der Schnittbilder zu visualisieren. Dabei kann zwischen Spannungen unterschieden werden, die unabhängig von einem intraokularen Druck, abhängig von einem intraokularen Druck und bedingt durch die Verformung der Hornhaut im Material der Hornhaut vorliegen. Diese Unterscheidung wird dadurch möglich, dass durch die Aufnahme der Schnittbilder Spannungen der unverformten Hornhaut und nachfolgend Spannungen der verformten Hornhaut aufgenommen und visualisiert werden können. Je nach Art, Stärke, Richtung oder Verteilung der Spannungen in den Schnittbildern der Hornhaut, kann der intraokulare Druck unter Berücksichtigung der Spannung korrigiert werden. Eine Korrektur des intraokularen Drucks kann insbesondere durch einen Vergleich eines Verhältnisses der Spannungen der unverformten und der verformten Hornhaut an einem definierten Punkt bzw. einer Position der verformten Hornhaut erfolgen. In einem weiteren Schritt des Verfahrens kann vorgesehen sein, die visualisierten Spannungen mit in einer Datenbank, gespeicherten, visualisierten Spannungen zu vergleichen, um den intraokularen Druck zu korrigieren. So kann für die in der Datenbank gespeicherten Werte ein objektiver Augeninnendruck oder auch ein entsprechender Korrekturwert bekannt sein, so dass der objektive intraokulare Druck des gemessenen Auges unter Berücksichtigung der Spannungen der Hornhaut abgeleitet werden kann.

Als ein Schnittbild kann dann jeweils eine spannungsoptische Darstellung der Hornhaut verwendet werden. Eine spannungsoptische Darstellung ermöglicht eine einfache Visualisierung einer Spannungsverteilung in lichtdurchlässigen Körpern, wobei eine Verteilung und Größe der mechanischen Spannung an allen Stellen der Hornhaut, oder auch in anderen lichtdurchlässigen Bereichen des Auges, einfach dargestellt und mittels Bildverarbeitung ausgewertet werden kann. Insbesondere können dabei in der Ebene des Schnittbildes auftretende Spannungen visualisiert werden. Quer zur Ebene des Schnittbildes verlaufende Spannungen werden dann nicht berücksichtigt, wobei dies zur Korrektur des intraokularen Drucks auch nicht zwingend notwendig ist.

Besonders einfach kann aus Spannungslinien der spannungsoptischen Darstellung die weitere Struktur- und/oder Materialeigenschaft der Hornhaut abgeleitet werden. Die Spannungslinien sind besonders gut visuell erkennbar und auch wird es dann möglich zwischen der weiteren Struktur- und der Materialeigenschaft der Hornhaut zu unterscheiden. Bei den Spannungslinien kann zwischen Isochromaten und Isoklinen unterschieden werden, wobei die Isochromaten Spannungslinien mit konstanter Hauptspannungsdifferenz sind und die Isoklinen Spannungstrajektorien der Hornhaut unter einer gegebenen Belastung repräsentieren. So können aus einer Vielzahl von während einer Verformung der Hornhaut gewonnen Schnittbildern, sich aufgrund der durch den Luftstoß bedingten Belastung der Hornhaut verändernde Spannungslinien und aufgrund einer Gestalt der Hornhaut selbst in dieser vorliegende Spannungslinien, welche sich relativ zur Hornhaut nicht wesentlich verändern, unterschieden werden. Dazu kann das Analysesystem in Art eines Polariskops ausgebildet sein, wobei das Beobachtungssystem dann eine Beleuchtungseinrichtung und eine Kameraeinrichtung umfassen kann, die jeweils einen Polarisator aufweisen.

Um neben der Steifigkeit der Hornhaut eine Elastizität als eine weitere Materialeigenschaft der Hornhaut bei der Messung zu berücksichtigen, kann eine Lichtstreuung der Hornhaut aus einem Schnittbild der Hornhaut abgeleitet werden, wobei aus der Lichtstreuung eines einzelnen Schnittbildes als eine Materialeigenschaft die Elastizität der Hornhaut abgeleitet wird. Eine optische Trübung der Hornhaut kann als ein Indikator für eine Materialalterung der Hornhaut verwendet werden, wobei aus einem Alter der Hornhaut Rückschlüsse über deren Elastizität gezogen werden können. So ist eine Hornhaut bei fortschreitender Trübung und somit erhöhter Lichtstreuung vergleichsweise weniger elastisch als eine Hornhaut mit einer geringeren Lichtstreuung. Die Elastizität der Hornhaut kann in diesem Fall als ein individueller Elastizitätsmodul des gemessenen Auges berücksichtigt werden.

Die Messung kann noch weiter dadurch verbessert werden, dass unterschiedlichen Bereichen der Hornhaut jeweils voneinander abweichende Materialeigenschaften zugeordnet werden. So können, bei angenommener, gleicher Dicke der Hornhaut die Materialeigenschaften in unterschiedlichen Bereichen eines Querschnitts der Hornhaut oder in Bezug auf einen Oberflächenbereich der Hornhaut variieren bzw. voneinander verschieden sein.

Das erfindungsgemäße ophthalmologische Analysesystem zur Messung eines objektiven intraokularen Drucks in einem Auge umfasst eine Betätigungseinrichtung, mit der eine Hornhaut des Auges berührungsfrei verformt werden kann, wobei mit der Betätigungseinrichtung ein Luftstoß zur Verformung der Hornhaut auf das Auge aufgebracht werden kann, ein Beobachtungssystem, mit dem die Verformung der Hornhaut beobachtet und aufgenommen werden kann, wobei das Beobachtungssystem eine Kamera und eine Beleuchtungseinrichtung in einer Scheimpfluganordnung umfasst, wobei mit der Kamera Schnittbilder der unverformten und/oder verformten Hornhaut aufgenommen werden können, und eine Analyseeinrichtung, mit der aus den Schnittbildern der Hornhaut der objektive intraokulare Druck abgeleitet werden kann, wobei in der Analyseeinrichtung aus den Schnittbildern der Hornhaut eine Materialeigenschaft der Hornhaut ableitbar ist, wobei als eine Materialeigenschaft eine Steifigkeit der Hornhaut ableitbar ist, wobei eine maximale Amplitude eines geometrischen Verformungsverlaufs der Hornhaut in Richtung einer Sehachse des Auges oder einer Geräteachse des Analysesystems aus den Schnittbildern der Hornhaut ableitbar ist, wobei eine Funktion der Steifigkeit für einen gemessenen subjektiven intraokularen Druck und die gemessene maximale Amplitude einer Datenbank des ophthalmologischen Analysesystems entnehmbar ist, und wobei der objektive intraokulare Druck unter Berücksichtigung der Steifigkeit in der Hornhaut ableitbar ist. Hinsichtlich der sich aus dem erfindungsgemäßen Analysesystem ergebenden vorteilhaften Wirkungen wird auf die Beschreibung des erfindungsgemäßen ophthalmologischen Analyseverfahrens verwiesen.

Weitere vorteilhafte Ausführungsformen des Analysesystems ergeben sich aus den Merkmalsbeschreibungen der auf den Verfahrensanspruch 1 rückbezogenen Unteransprüche.

Nachfolgend wird eine bevorzugte Ausführungsform der Erfindung unter Bezugnahme auf die beigefügten Zeichnungen näher erläutert.

Es zeigen:
- **Fig. 1a** bis **1e**:: eine Verformung einer Hornhaut eines Auges während einer Messung in einer Längsschnittansicht;
- **Fig. 2:**: eine Diagrammdarstellung von Pumpendruck und -zeit während einer Messung;
- **Fig. 3:**: eine Diagrammdarstellung von gemessenem intraokularen Druck und Verformung der Hornhaut;
- **Fig. 4a** bis 4b:: eine Visualisierung von Spannungen im Material der Hornhaut des Auges.

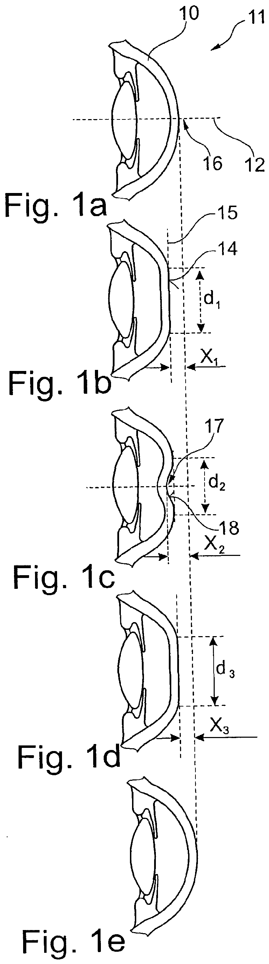

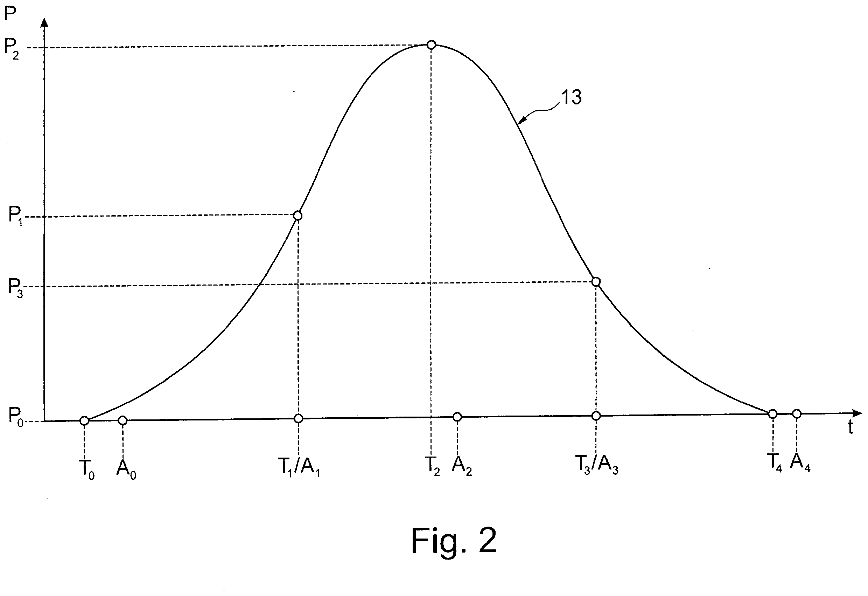

Die **Fig. 1a** bis **1e** zeigen ausgewählte Verformungszustände einer Hornhaut 10 eines Auges 11 während einer einzelnen Messung eines Augeninnendrucks durch ein hier nicht dargestelltes Analysesystem. Die Darstellungen sind jeweils Längsschnittdarstellungen entlang einer Sehachse 12 des Auges 11. **Fig. 2** ist eine Diagrammdarstellung mit einer Zeit t auf der Abzissenachse und einem Pumpendruck p auf der Ordinatenachse. Der Pumpendruck verläuft unabhängig vom Einsatz eines hier nicht dargestellten Beobachtungssystems bzw. einer Scheimpflugkamera mit einer Spaltbeleuchtungseinrichtung in Art einer symmetrischen Glockenkurve 13, beginnend mit einem Druck P₀ bei einem Startpunkt T₀ der Pumpe bis hin zu einem maximalen Pumpendruck P₂ zum Zeitpunkt T₂ und wieder abfallend bis hin zu dem Pumpendruck P₀ in einem Endpunkt T₄. Der durch den Start der Pumpe bei T₀ abgegebene Luftstoß auf die Hornhaut 10 führt zu einer ersten, mittels des Beobachtungssystems aufnehmbaren Verformung der Hornhaut 10 unmittelbar nach dem Zeitpunkt A₀. **Fig. 1a** repräsentiert die Gestalt der noch nicht verformten Hornhaut 10 zum Zeitpunkt A₀. Mit steigendem Pumpendruck kommt es zum Zeitpunkt A₁ zu einer vollständigen Applanation der Hornhaut 10 gemäß **Fig. 1b**, wobei wie hier dargestellt, eine Applanationsfläche 14 mit einem Durchmesser d₁ ausgebildet wird, die im Wesentlichen eben ist und in einer Applanationsebene 15 liegt. Die Hornhaut ist dann um ein Maß X₁ vom Apex 16 der Hornhaut 10 entfernt bzw. eingedrückt. Optional und nicht notwendigerweise kann bei diesem sogenannten ersten Applanationspunkt zum Zeitpunkt A₁ ein Pumpendruck P₁ zum übereinstimmenden Zeitpunkt T₁ ermittelt werden. Nach Erreichen des Pumpendrucks P₂ kommt es zu einer maximalen Verformung der Hornhaut 10 zum Zeitpunkt A₂ entsprechend der Darstellung in **Fig. 1c**. Ein eine maximale Verformung bestimmender Punkt 17 ist dabei um ein Maß X₂ vom Apex 16 der Hornhaut 10 entfernt. Es handelt sich in diesem Fall also um eine maximale Auslenkung einer Amplitude der Verformung. Bei dieser maximalen Amplitude der Verformung wird ein Durchmesser d₂ einer konkaven Verformungsfläche 18 ausgebildet und erfasst. Der Durchmesser d₂ ist durch einen Abstand von zwei gegenüberliegenden Punkten einer Längsschnittebene der Hornhaut 10 definiert, wobei die Punkte jeweils die dem Analysesystem am nächsten zugewandten Punkte der Hornhaut 10 repräsentieren. Nachfolgend kommt es zu einer Rückbewegung bzw. einem Ausschwingen der Hornhaut 10, wobei zum Zeitpunkt A₃ der sogenannte zweite Applanationspunkt gemäß Darstellung in **Fig. 1d** erreicht wird. Hier wird ebenfalls ein Durchmesser d₃ sowie ein Abstand X₃ erfasst. Auch ist es optional möglich einen Pumpendruck P₃ zum übereinstimmenden Zeitpunkt T₃ zu bestimmen. Nach dem Rückgang des Pumpendrucks auf den Ursprungswert P₀ zum Zeitpunkt T₄ gelangt die Hornhaut 10 zum Zeitpunkt A₄ ebenfalls wieder in ihre in **Fig. 1e** dargestellte Ausgangslage. Entsprechend der vorangehenden Beschreibung einer einzelnen Messung eines intraokularen Drucks eines Auges werden die in den **Fig. 1a** bis **1e** dargestellten Verformungszustände der Hornhaut 10, welche durch die jeweiligen mit A₀ bis A₄ gekennzeichneten Zeitpunkte charakterisiert sind, ermittelt. Dabei werden insbesondere unabhängig von einem Pumpendruck p Zeitabstände der betreffenden Zeitpunkte A₀ bis A₄ sowie die Maße bzw. Eindrücktiefen X₁, X₂ und X₃ erfasst, wobei aus diesen Kenngrößen eine Steifigkeit der Hornhaut 10 abgeleitet wird. Ein gemessener intraokularer Druck wird dann durch einen durch die Steifigkeit der Hornhaut bestimmten Wert korrigiert, sodass ein objektiver intraokularer Druck als ein Ergebnis der Messung ausgegeben wird.

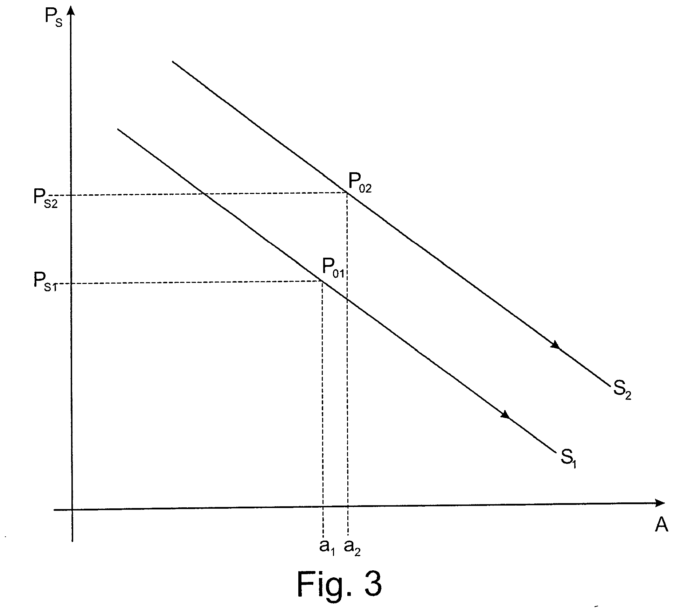

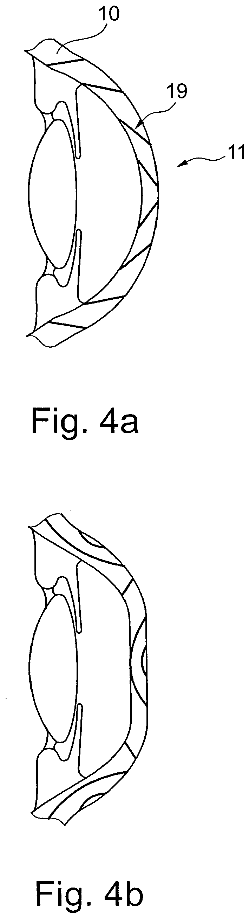

**Fig. 3** zeigt eine Diagrammdarstellung mit einem subjektiven, gemessenen intraokularen Druck auf der Ordinatenachse und einer Auslenkung einer Amplitude einer maximalen Verformung der Hornhaut 10 auf der Abzissenachse. Bei beispielsweise einem subjektiven intraokularen Druck auf Pₛ₁ und einer Amplitude a₁, welche dem Abstand X₂ entspricht, ergibt sich eine Steifigkeit S₁ als eine im Wesentlichen lineare Funktion mit negativer Steigung. Die Steifigkeit S₁ kann jedoch auch von einer linearen Funktion abweichen und als eine Linie mit einem vergleichsweise großen Krümmungsradius ausgebildet sein. Ein objektiver intraokularer Druck Pₒ₁ kann von der durch die Steifigkeit S₁ definierten Geraden als eine Variable entnommen werden. Analog dazu ergibt sich bei einem Druck Pₛ₂ und einer Auslenkung a₂ eine Parallelverschiebung der Geraden mit einer Steifigkeit S₂, woraus wieder ein objektiver intraokularer Druck Pₒ₂ abgeleitet werden kann. Alternativ, jedoch nicht unter die Ansprüche fallend, können auch anstelle der Amplituden a₁ und a₂ die Durchmesser d₁ und d₂ in dem Diagramm eingesetzt und analog verwendet werden. Die **Fig. 4a** bis **4b** zeigen die Verformungszustände der Hornhaut 10 des Auges 11 analog den **Fig. 1a** und **1b**. Im Unterschied dazu sind bei den **Fig. 4a** und **4b** Spannungen im Material der Hornhaut visualisiert. So sind insbesondere Spannungslinien 19 im Material der Hornhaut 10 sichtbar, die Hauptspannungen längs und quer der Sehachse 12 repräsentieren. Die **Fig. 4a** zeigt somit Spannungen im Auge 11 in einem Ruhezustand der Hornhaut 10 und die **Fig. 4b** Spannungen im Auge 11 der verformten Hornhaut 10, die sich von den Spannungen im Ruhezustand wesentlich unterscheiden. Ein Vergleich der Spannung auf Basis der Spannungslinien 19 ermöglicht somit eine Definition einer Struktur- und/oder Materialeigenschaft der Hornhaut, die zur Korrektur eines gemessenen intraokularen Drucks und damit zur Ableitung eines objektiven intraokularen Drucks herangezogen werden kann.

## Patentansprüche

1. Ophthalmologisches Analyseverfahren zur Messung eines objektiven intraokularen Drucks in einem Auge (11) mit einem Analysesystem, gebildet aus einer Betätigungseinrichtung, mit der eine Hornhaut (10) des Auges berührungsfrei verformt wird, wobei mit der Betätigungseinrichtung ein Luftstoß zur Verformung der Hornhaut auf das Auge aufgebracht wird, einem Beobachtungssystem, mit dem die Verformung der Hornhaut beobachtet und aufgenommen wird, wobei das Beobachtungssystem eine Kamera und eine Beleuchtungseinrichtung in einer Scheimpfluganordnung umfasst, wobei mit der Kamera Schnittbilder der unverformten und verformten Hornhaut aufgenommen werden, und einer Analyseeinrichtung, mit der aus den Schnittbildern der Hornhaut der objektive intraokulare Druck abgeleitet wird, wobei in der Analyseeinrichtung aus den Schnittbildern der Hornhaut eine Materialeigenschaft der Hornhaut abgeleitet wird, wobei als eine Materialeigenschaft eine Steifigkeit der Hornhaut abgeleitet wird, wobei eine maximale Amplitude eines geometrischen Verformungsverlaufs der Hornhaut (10) in Richtung einer Sehachse (12) des Auges oder einer Geräteachse des Analysesystems aus den Schnittbildern der Hornhaut abgeleitet wird, wobei eine Funktion der Steifigkeit für einen gemessenen subjektiven intraokularen Druck und die gemessene maximale Amplitude einer Datenbank des ophthalmologischen Analysesystems entnommen wird, wobei der objektive intraokulare Druck unter Berücksichtigung der Steifigkeit der Hornhaut abgeleitet wird.

2. Analyseverfahren nach Anspruch 1,
**dadurch gekennzeichnet,**
**dass** eine weitere, vom intraokularen Druck unabhängige Materialeigenschaft der Hornhaut (10) abgeleitet wird.

3. Analyseverfahren nach Anspruch 1 oder 2,
**dadurch gekennzeichnet,**
**dass** ein Pumpendruck zur Erzeugung des Luftstoßes relativ zu einer Zeitdauer desselben in Form einer Glockenkurve (13) verläuft.

4. Analyseverfahren nach einem der vorangehenden Ansprüche,
**dadurch gekennzeichnet,**
**dass** ein maximaler Pumpendruck zur Erzeugung des Luftstoßes bei vorangehenden und nachfolgenden Messungen gleich ist.

5. Analyseverfahren nach einem der vorangehenden Ansprüche,
**dadurch gekennzeichnet,**
**dass** ein Pumpendruck zur Erzeugung des Luftstoßes bei Erreichen eines Applanationspunktes der Hornhaut (10) gemessen wird.

6. Analyseverfahren nach einem der vorangehenden Ansprüche,
**dadurch gekennzeichnet,**
**dass** ein Zeitabschnitt zwischen Beginn und Ende der Verformung der Hornhaut (10) gemessen wird, wobei der Zeitabschnitt zur Ableitung der Materialeigenschaft gemessen wird.

7. Analyseverfahren nach Anspruch 6,
**dadurch gekennzeichnet,**
**dass** eine Geschwindigkeit der bewegten Hornhaut (10) gemessen wird, wobei die Geschwindigkeit zur Ableitung der Materialeigenschaft gemessen wird.

8. Analyseverfahren nach einem der vorangehenden Ansprüche,
**dadurch gekennzeichnet,**
**dass** ein Entfernungsmaß (X₂) einer maximalen Verformung der Hornhaut (10) relativ zum Apex (16) der Hornhaut aus den Schnittbildern der Hornhaut abgeleitet wird, wobei das Entfernungsmaß (X₂) zur Ableitung der Materialeigenschaft aus den Schnittbildern abgeleitet wird.

9. Analyseverfahren nach einem der vorangehenden Ansprüche,
**dadurch gekennzeichnet,**
**dass** eine Krümmung der unverformten und/oder verformten Hornhaut (10) aus den Schnittbildern der Hornhaut abgeleitet wird, wobei die Krümmung zur Ableitung der Materialeigenschaft aus den Schnittbildern abgeleitet wird.

10. Analyseverfahren nach einem der vorangehenden Ansprüche,
**dadurch gekennzeichnet,**
**dass** bei Erreichen eines Applanationspunktes der Hornhaut (10) ein Durchmesser einer ebenen Applanationsfläche gemessen wird, wobei der Durchmesser zur Ableitung der Materialeigenschaft gemessen wird.

11. Analyseverfahren nach einem der vorangehenden Ansprüche,
**dadurch gekennzeichnet,**
**dass** als eine weitere Materialeigenschaft ein Schubmodul (G) der Hornhaut (10) abgeleitet wird.

12. Analyseverfahren nach einem der Ansprüche 1 bis 10,
**dadurch gekennzeichnet,**
**dass** als Materialeigenschaft eine nichtlineare Steifigkeit der Hornhaut (10) abgeleitet wird.

13. Analyseverfahren nach einem der vorangehenden Ansprüche,
**dadurch gekennzeichnet,**
**dass** als eine weitere Materialeigenschaft eine Spannung im Material der Hornhaut (10) abgeleitet wird, wobei die Spannung aus einer spannungsoptischen Darstellung der Hornhaut abgeleitet wird.

14. Analyseverfahren nach einem der vorangehenden Ansprüche,
**dadurch gekennzeichnet,**
**dass** eine Lichtstreuung der Hornhaut (10) aus einem Schnittbild der Hornhaut abgeleitet wird, wobei aus der Lichtstreuung eines einzelnen Schnittbildes als eine weitere Materialeigenschaft eine Elastizität der Hornhaut abgeleitet wird.

15. Analyseverfahren nach einem der Ansprüche 11 bis 14,
**dadurch gekennzeichnet,**
**dass** unterschiedlichen Bereichen der Hornhaut (10) jeweils voneinander abweichende Materialeigenschaften zugeordnet werden.

16. Ophthalmologisches Analysesystem zur Messung eines objektiven intraokularen Drucks in einem Auge (11), umfassend eine Betätigungseinrichtung, mit der eine Hornhaut (10) des Auges berührungsfrei verformt werden kann, wobei mit der Betätigungseinrichtung ein Luftstoß zur Verformung der Hornhaut auf das Auge aufgebracht werden kann, ein Beobachtungssystem, mit dem die Verformung der Hornhaut beobachtet und aufgenommen werden kann, wobei das Beobachtungssystem eine Kamera und eine Beleuchtungseinrichtung in einer Scheimpfluganordnung umfasst, wobei mit der Kamera Schnittbilder der unverformten und verformten Hornhaut aufgenommen werden können, und eine Analyseeinrichtung, dazu eingerichtet, aus den Schnittbildern der Hornhaut den objektiven, intraokularen Druck abzuleiten, wobei die Analyseeinrichtung weiter dazu eingerichtet ist, aus den Schnittbildern der Hornhaut eine Materialeigenschaft der Hornhaut abzuleiten, wobei als eine Materialeigenschaft eine Steifigkeit der Hornhaut ableitbar ist wobei die Analyseeinrichtung dazu eingerichtet ist, eine maximale Amplitude eines geometrischen Verformungsverlaufs der Hornhaut (10) in Richtung einer Sehachse (12) des Auges oder einer Geräteachse des Analysesystems aus den Schnittbildern der Hornhaut abzuleiten, wobei die Analyseeinrichtung dazu eingerichtet ist, eine Funktion der Steifigkeit für einen gemessenen subjektiven intraokularen Druck und die gemessene maximale Amplitude einer Datenbank des ophthalmologischen Analysesystems zu entnehmen, wobei die Analyseeinrichtung dazu eingerichtet ist, den objektiven, intraokularen Druck unter Berücksichtigung der Steifigkeit der Hornhaut abzuleiten.

## Claims

1. An ophthalmological analysis method for measuring an objective intraocular pressure in an eye (11) with an analysis system consisting of an actuating device with which a cornea (10) of the eye is deformed in a contactless manner, wherein an air puff is applied to the eye by means of the actuating device to deform the cornea, an observation system with which the deformation of the cornea is observed and recorded, wherein the observation system comprises a camera and an illumination device in a Scheimpflug arrangement, wherein sectional images of the undeformed and deformed cornea are taken with the camera, and an analysis device with which the objective intraocular pressure is derived from the sectional images of the cornea, wherein a material characteristic of the cornea is derived from the sectional images of the cornea in the analysis device, wherein a stiffness of the cornea is derived as a material characteristic, wherein a maximum amplitude of a geometric deformation course of the cornea (10) in the direction of a visual axis (12) of the eye or of a device axis of the analysis system is derived from the sectional images of the cornea, wherein a function of the stiffness for a measured subjective intraocular pressure and the measured maximum amplitude are taken from a data base of the ophthalmological analysis system, wherein the objective intraocular pressure is derived taking into account the stiffness of the cornea.

2. The analysis method as recited in claim 1,
**characterized in that**
a further material characteristic of the cornea (10) that is independent of the intraocular pressure is derived.

3. The analysis method as recited in claim 1 or 2,
**characterized in that**
a pump pressure for producing an air puff follows the form of a bell curve (13) relative to a duration thereof.

4. The analysis method as recited in any one of the preceding claims,
**characterized in that**
a maximum pump pressure for producing the air puff is the same in preceding and following measurements.

5. The analysis method as recited in any one of the preceding claims,
**characterized in that**
a pump pressure for producing the air puff is measured when an applanation point of the cornea (10) is reached.

6. The analysis method as recited in any one of the preceding claims,
**characterized in that**
a time period between the start and end of the deformation of the cornea (10) is measured, wherein the time period is measured for the purpose of deriving the material characteristic.

7. The analysis method as recited in claim 6,
**characterized in that**
a speed of the moved cornea (10) is measured, wherein the speed is measured for the purpose of deriving the material characteristic.

8. The analysis method as recited in any one of the preceding claims,
**characterized in that**
a distance measure (X₂) of a maximum deformation of the cornea (10) relative to the apex (16) of the cornea is derived from the sectional images of the cornea, wherein the distance measure (X₂) is derived for the purpose of deriving the material characteristic from the sectional images.

9. The analysis method as recited in any one of the preceding claims,
**characterized in that**
a curvature of the undeformed and/or deformed cornea (10) is derived from the sectional images of the cornea, wherein the curvature is derived for the purpose of deriving the material characteristic from the sectional images.

10. The analysis method as recited in any one of the preceding claims,
**characterized in that**
a diameter of a flat applanation area is measured when an applanation point of the cornea (10) is reached, wherein the diameter is measured for the purpose of deriving the material characteristic.

11. The analysis method as recited in any one of the preceding claims,
**characterized in that**
a shear modulus (G) of the cornea (10) is derived as a further material characteristic.

12. The analysis method as recited in any one of claims 1 to 10,
**characterized in that**
a non-linear stiffness of the cornea (10) is derived as material characteristic.

13. The analysis method as recited in any one of the preceding claims,
**characterized in that**
a stress in the material of the cornea (10) is derived as a further material characteristic, wherein the stress is derived from a photoelastic representation of the cornea.

14. The analysis method as recited in any one of the preceding claims,
**characterized in that**
a light scattering of the cornea (10) is derived from a sectional image of the cornea, wherein an elasticity of the cornea is derived as a further material characteristic from the light scattering of an individual sectional image.

15. The analysis method as recited in any one of claims 11 to 14,
**characterized in that**
material characteristics that differ from each other are assigned to each different region of the cornea (10).

16. An ophthalmological analysis system for measuring an objective intraocular pressure in an eye (11), said ophthalmological analysis system comprising an actuating device with which a cornea (10) of the eye can be deformed in a contactless manner, wherein an air puff can be applied to the eye by means of the actuating device to deform the cornea, an observation system with which the deformation of the cornea can be observed and recorded, wherein the observation system comprises a camera and an illumination device in a Scheimpflug arrangement, wherein sectional images of the undeformed and deformed cornea can be taken with the camera, and an analysis device designed to derive the objective intraocular pressure from the sectional images of the cornea, wherein the analysis device is furthermore designed to derive a material characteristic of the cornea from the sectional images of the cornea, wherein a stiffness of the cornea can be derived as a material characteristic, wherein the analysis device is designed to derive a maximum amplitude of a geometric deformation course of the cornea (10) in the direction of a visual axis (12) of the eye or of a device axis of the analysis system from the sectional images of the cornea, wherein the analysis device is designed to take a function of the stiffness for a measured subjective intraocular pressure and the measured maximum amplitude from a data base of the ophthalmological analysis system, wherein the analysis device is designed to derive the objective intraocular pressure taking into account the stiffness of the cornea.

## Revendications

1. Procédé d'analyse ophtalmologique pour mesurer une pression intraoculaire objective dans un œil (11) au moyen d'un système d'analyse composé d'un moyen d'actionnement au moyen duquel une cornée (10) de l'œil est déformée sans contact, dans lequel une bouffée d'air est appliquée sur l'œil au moyen du moyen d'actionnement pour déformer la cornée, d'un système d'observation au moyen duquel la déformation de la cornée est observée et enregistrée, dans lequel le système d'observation comprend une caméra et un moyen d'éclairage dans une disposition de Scheimpflug, dans lequel des images en coupe de la cornée non déformée et déformée sont prises avec la caméra, et d'un moyen d'analyse au moyen duquel la pression intraoculaire objective est dérivée des images en coupe de la cornée, dans lequel une propriété du matériau de la cornée est dérivée des images en coupe de la cornée dans le moyen d'analyse, dans lequel une rigidité de la cornée est dérivée comme propriété du matériau, dans lequel une amplitude maximale d'un déroulement géométrique de la déformation de la cornée (10) dans la direction d'un axe visuel (12) de l'œil ou d'un axe du dispositif du système d'analyse est dérivée des images en coupe de la cornée, dans lequel une fonction de la rigidité pour une pression intraoculaire subjective mesurée et l'amplitude maximale mesurée sont prises d'une base de données du système d'analyse ophtalmologique, dans lequel la pression intraoculaire objective est dérivée en tenant compte de la rigidité de la cornée.

2. Procédé d'analyse selon la revendication 1,
**caractérisé en ce**
**qu'**une autre propriété du matériau de la cornée (10) qui est indépendante de la pression intraoculaire est dérivée.

3. Procédé d'analyse selon la revendication 1 ou 2,
**caractérisé en ce**
**qu'**une pression de pompe pour générer la bouffée d'air suit la forme d'une courbe en cloche (13) par rapport à une durée de celle-ci.

4. Procédé d'analyse selon l'une quelconque des revendications précédentes,
**caractérisé en ce**
**qu'**une pression de pompe maximale pour générer la bouffée d'air est la même dans des mesures précédentes et suivantes.

5. Procédé d'analyse selon l'une quelconque des revendications précédentes,
**caractérisé en ce**
**qu'**une pression de pompe pour générer la bouffée d'air est mesurée lorsqu'un point d'aplanation de la cornée (10) est atteint.

6. Procédé d'analyse selon l'une quelconque des revendications précédentes,
**caractérisé en ce**
**qu'**une période entre le début et la fin de la déformation de la cornée (10) est mesurée, dans lequel la période est mesurée pour dériver les propriétés du matériau.

7. Procédé d'analyse selon la revendication 6,
**caractérisé en ce**
**qu'**une vitesse de la cornée (10) bougée est mesurée, dans lequel la vitesse est mesurée pour dériver la propriété du matériau.

8. Procédé d'analyse selon l'une quelconque des revendications précédentes,
**caractérisé en ce**
**qu'**une mesure de distance (X₂) d'une déformation maximale de la cornée (10) par rapport à l'apex (16) de la cornée (10) est dérivée des images en coupe de la cornée, dans lequel la mesure de distance (X₂) est dérivée des images en coupe pour dériver la propriété du matériau.

9. Procédé d'analyse selon l'une quelconque des revendications précédentes,
**caractérisé en ce**
**qu'**une courbure de la cornée (10) non déformée et/ou déformée est dérivée des images en coupe de la cornée, dans lequel la courbure est dérivée des images en coupe pour dériver la propriété du matériau.

10. Procédé d'analyse selon l'une quelconque des revendications précédentes,
**caractérisé en ce**
**qu'**un diamètre d'une face d'aplanation plate est mesuré lorsqu'un point d'aplanation de la cornée (10) est atteint, dans lequel le diamètre est mesuré pour dériver la propriété du matériau.

11. Procédé d'analyse selon l'une quelconque des revendications précédentes,
**caractérisé en ce**
**qu'**un module de cisaillement (G) de la cornée (10) est dérivé comme autre propriété du matériau.

12. Procédé d'analyse selon l'une quelconque des revendications 1 à 10,
**caractérisé en ce**
**qu'**une rigidité non linéaire de la cornée (10) est dérivée comme propriété du matériau.

13. Procédé d'analyse selon l'une quelconque des revendications précédentes,
**caractérisé en ce**
**qu'**une tension dans le matériau de la cornée (10) est dérivée comme autre propriété du matériau, dans lequel la tension est dérivée d'une représentation photoélastique de la cornée.

14. Procédé d'analyse selon l'une quelconque des revendications précédentes,
**caractérisé en ce**
**qu'**une diffusion de la lumière de la cornée (10) est dérivée d'une image en coupe de la cornée, dans lequel une élasticité de la cornée est dérivée comme autre propriété du matériau de la diffusion de la lumière d'une image en coupe individuelle.

15. Procédé d'analyse selon l'une quelconque des revendications 11 à 14,
**caractérisé en ce que**
des propriétés du matériau qui diffèrent les unes des autres sont attribuées à chacune des différentes zones de la cornée (10).

16. Système d'analyse ophtalmologique pour mesurer une pression intraoculaire objective dans un œil (11), ledit système d'analyse ophtalmologique comprenant un moyen d'actionnement au moyen duquel une cornée (10) de l'œil peut être déformée sans contact, dans lequel une bouffée d'air peut être appliquée sur l'œil au moyen du moyen d'actionnement pour déformer la cornée, un système d'observation au moyen duquel la déformation de la cornée peut être observée et enregistrée, dans lequel le système d'observation comprend une caméra et un moyen d'éclairage dans une disposition de Scheimpflug, dans lequel des images en coupe de la cornée non déformée et déformée peuvent être prises avec la caméra, et un moyen d'analyse conçu pour dériver la pression intraoculaire objective des images en coupe de la cornée, dans lequel le moyen d'analyse en plus est conçu pour dériver une propriété du matériau de la cornée des images en coupe de la cornée, dans lequel une rigidité de la cornée peut être dérivée comme propriété du matériau, dans lequel le moyen d'analyse est conçu pour dériver une amplitude maximale d'un déroulement géométrique de la déformation de la cornée (10) dans la direction d'un axe visuel (12) de l'œil ou d'un axe du dispositif du système d'analyse des images en coupe de la cornée, dans lequel le moyen d'analyse est conçu pour prendre une fonction de la rigidité pour une pression intraoculaire subjective mesurée et l'amplitude maximale mesurée d'une base de données du système d'analyse ophtalmologique, dans lequel le moyen d'analyse est conçu pour dériver la pression intraoculaire objective en tenant compte de la rigidité de la cornée.
